# EUROPÄISCHE PATENTSCHRIFT

(11) **EP 2 242 432 B1**
(45) Veröffentlichungstag und Bekanntmachung des Hinweises auf die Patenterteilung: **05.03.2014**
(21) Anmeldenummer: 08872506.4
(22) Anmeldetag: 26.11.2008
(51) Int. Cl.: A61B 17/17, A61B 17/70, A61B 17/80, A61B 17/86, A61B 19/02

(54) **MAGAZIN ZUR AUFNAHME MINDESTENS EINER KNOCHENSCHRAUBE UND KNOCHENPLATTE MIT EINEM DERARTIGEN MAGAZIN**
MAGAZINE FOR RECEIVING AT LEAST ONE BONE SCREW AND BONE PLATE HAVING SUCH A MAGAZINE
MAGASIN POUR RECEVOIR AU MOINS UNE VIS D'OSTEOSYNTHESE, ET PLAQUE D OSTEOSYNTHESE POURVUE D UN TEL MAGASIN

(30) Priorität: 21.02.2008 DE 102008010333
(43) Veröffentlichungstag der Anmeldung: 27.10.2010
(73) Patentinhaber: Aesculap AG, 78532 Tuttlingen (DE)
(72) Erfinder: BEGER, Jens, 78532 Tuttlingen (DE); SCHUMACHER, Jörg, 14513 Teltow (DE); LINKE, Ralph, 78256 Steisslingen (DE)
(74) Vertreter: Hoeger, Stellrecht & Partner Patentanwälte
(86) Internationale Anmeldenummer: PCT/EP2008/010011
(87) Internationale Veröffentlichungsnummer: WO 2009/103323

(56) Entgegenhaltungen:
- WO-A-2007/070196
- US-A1- 2004 204 717
- US-A1- 2006 229 618

## Beschreibung

Die Erfindung betrifft eine Knochenplatte mit einem darauf aufgesetzten Magazin zur Aufnahme von mindestens einer Knochenschraube.

Knochenplatten weisen in der Regel mehrere Einschrauböffnungen auf, durch die Knochenschrauben hindurchgeschraubt werden, mit deren Hilfe die Knochenplatten an Knochenteilen festgelegt werden. Das Einschrauben dieser Knochenschrauben kann schwierig sein, da die Operationsgebiete häufig schlecht zugänglich sind und da die Knochenschrauben meistens relativ kurz sind. Es besteht daher die Gefahr von Fehlorientierungen der Knochenschrauben und auch die Gefahr, dass eine Knochenschraube im Operationsgebiet herabfällt und dann mühsam aufgefunden werden muss.

Es ist bekannt, auf Knochenplatten Führungshülsen aufzusetzen, um beim Einschrauben der Knochenschrauben diesen eine Führung zu geben. In der US 2006/0149250 A1 ist beispielsweise eine Knochenplatte beschrieben, bei der in die Einschrauböffnungen hülsenförmige Führungskörper eingeschraubt werden können, die nach dem Einschrauben der Knochenschrauben einzeln wieder aus den Einschrauböffnungen entfernt werden müssen. Diese Konstruktion setzt voraus, dass die Einschrauböffnungen ein Einschraubgewinde für die Führungshülsen aufweisen, es ist also ein relativ großer apparativer Aufwand notwendig, außerdem ist das Entfernen der Führungshülsen umständlich, da bei jeder Einschrauböffnung eine entsprechende Führungshülse herausgeschraubt werden muss.

In der US 2005/0177163 A1 ist eine Schablone beschrieben, die mit Hilfe eines Handgriffes auf eine Knochenplatte aufgesetzt werden kann, diese Schablone weist Führungsöffnungen für Knochenschrauben auf. Nach dem Einschrauben der Knochenschrauben kann die Schablone insgesamt mittels eines Handgriffes wieder von der Knochenplatte abgenommen werden. Auch bei dieser bekannten Konstruktion ergeben sich bei der Anwendung Schwierigkeiten, da die Knochenschrauben in die Führungsöffnungen der Schablone eingesetzt werden müssen, auch dies kann an schwer zugänglichen Operationsstellen zu Problemen führen.

In der W02007/070196 A2 ist ein Magazin beschrieben, das auf eine Knochenplatte lösbar aufgesetzt werden kann. Es verfügt jedoch über keine Fixiervorrichtung zur lösbaren Festlegung einer Knochenschraube in der Aufnahmekammer des Magazins.

In der US 2006/0229618 A1 und in der US 2004/0204717 A1 sind weitere Magazine beschrieben.

Es ist Aufgabe der Erfindung, ein Magazin zur Aufnahme von mindestens einer Knochenschraube vorzusehen, mit welchem das Einschrauben einer Knochenschraube in eine Knochenplatte erleichtert werden kann.

Diese Aufgabe wird erfindungsgemäß gelöst durch eine Knochenplatte mit einem lösbar darauf festgelegten Magazin zur Aufnahme von mindestens einer Knochenschraube mit mindestens einer von der Oberseite zur Unterseite des Magazins durchgehenden Aufnahmekammer, in der eine Knochenschraube derart angeordnet ist, dass ihre distale Spitze auf eine Einschrauböffnung der Knochenplatte gerichtet ist und ihr proximales Ende von der Oberseite des Magazins für ein Einschraubwerkzeug zugänglich ist, und mit einer Fixiervorrichtung zur lösbaren Festlegung der Knochenschraube in der Aufnahmekammer.

Durch das lösbare Aufsetzen eines Magazins auf die Knochenplatte wird die in der Aufnahmekammer des Magazins angeordnete Knochenschraube in einer Einschraubposition bereitgestellt, bei der sie auf die jeweilige Einschrauböffnung ausgerichtet ist und somit bereits an der Knochenplatte positioniert ist. Beim Einsetzen der Knochenplatte wird diese zusammen mit dem Magazin eingesetzt, so dass kein komplizierter Einführvorgang für eine Knochenschraube mehr notwendig ist. Sobald die Knochenplatte im Operationsgebiet in der gewünschten Weise positioniert ist, kann man einfach mittels eines Einschraubwerkzeuges die im Magazin in Einschraubposition gehaltene Knochenschraube in distaler Richtung, also in Richtung auf die Einschrauböffnung und den darunter angeordneten Knochen, vorschieben und dann durch die Einschrauböffnung hindurch in den Knochen einschrauben. Wenn in dieser Weise die Knochenschraube in die Knochenplatte eingeschraubt ist, kann das Magazin, das an der Knochenplatte lösbar festgelegt ist, entfernt werden.

Durch die Verwendung eines bereits die Knochenschraube halternden Magazins entfällt der umständliche Einführvorgang für die Knochenschraube, außerdem findet der Operateur die Knochenschraube bereits in der gewünschten Einschraubposition und Einschraubrichtung relativ zur Knochenplatte, so dass keinerlei Justiervorgänge mehr notwendig sind.

Die Aufnahmekammer kann insbesondere die Form eines Kanals aufweisen.

Besonders vorteilhaft ist es, wenn die Aufnahmekammer eine Längsführung für die Knochenschraube bildet, längs welcher die Knochenschraube beim Einschrauben in distaler Richtung durch die Einschrauböffnung hindurch vorschiebbar ist. Beim Einschrauben muss der Operateur daher in keiner Weise mehr auf die Einschraubrichtung achten, diese wird durch die Führung in der Längsführung des Magazins vorgegeben und aufrechterhalten.

Bei einer ersten bevorzugten Ausführungsform ist vorgesehen, dass die Knochenschraube im Reibsitz an der Innenwand der Aufnahmekammer anliegt und dadurch lösbar in der Aufnahmekammer gehalten ist. Der Reibsitz kann beim Einschrauben überwunden werden, der Reibsitz reicht aber aus, um die Knochenschraube beim Einsetzen der Knochenplatte in das Operationsgebiet in der vorbereiteten Einschraubposition zu halten.

Bei einer anderen bevorzugten Ausführungsform ist vorgesehen, dass die Fixiervorrichtung mindestens einen elastischen Rastvorsprung und mindestens einen mit diesem zusammenwirkenden Rastrücksprung umfasst. Zwischen Knochenschraube und Magazin ergibt sich somit eine elastische Rastverbindung, die vom Operateur beim Einschrauben überwunden werden kann.

Beispielsweise kann mindestens ein elastischer Rastvorsprung am Magazin in die Aufnahmekammer hineinragen und in einen Rücksprung an der Knochenschraube eintauchen.

Ein derartiger Rastvorsprung kann unmittelbar an dem Magazin selbst angeordnet sein oder aber auch an einem in die Aufnahmekammer eingesetzten Halteelement. Beispielsweise kann in die Aufnahmekammer eine Hülse eingesetzt sein, die in der Aufnahmekammer festgelegt ist und die ihrerseits einen elastischen Rastvorsprung für die Knochenschraube trägt. Dieses Halteelement kann an unterschiedliche Dimensionen der Knochenschraube angepasst sein, so dass ein Magazin für Knochenschrauben unterschiedlicher Dimensionen Verwendung finden kann.

Der Rastvorsprung am Magazin kann beispielsweise an einen Rastrücksprung in Form einer an der Knochenschraube vorgesehenen Umfangsnut eintauchen. Bei einer weiteren bevorzugten Ausführungsform ist vorgesehen, dass die Fixiereinrichtung einen Vorsprung an der Knochenschraube oder der Aufnahmekammer und einen Rücksprung an der Aufnahmekammer beziehungsweise der Knochenschraube aufweist, in den der Vorsprung eingreift, und dass der Vorsprung und/oder der Rücksprung nach Art eines Gewindes wendelförmig ausgebildet sind. Eine Festlegung erfolgt daher durch Einschrauben, und entsprechend erfolgt die Freigabe durch eine Drehbewegung der Knochenschraube gegenüber der Aufnahmekammer und damit ein Ausschrauben der Knochenschraube aus der Aufnahmekammer.

Insbesondere kann die Steigung des wendelförmigen Vorsprungs und/oder Rücksprungs der Steigung des Gewindes der Knochenschraube entsprechen, so dass das Ausschrauben aus der Aufnahme und damit die Freigabe aus der Aufnahmekammer gleichzeitig mit dem Einschrauben der Knochenschraube in den Knochen erfolgen kann.

Gemäß einer bevorzugten Ausführungsform werden der Vorsprung oder Rücksprung an der Knochenschraube zumindest teilweise von dem Gewinde der Knochenschraube gebildet. In diesem Falle ist es günstig, wenn der Kerndurchmesser der Knochenschraube im oberen Teil zunimmt, so dass dadurch in diesem oberen Gewindebereich eine Anlage an der Knochenplatte möglich wird, durch die die Knochenschraube dann die Knochenplatte gegen den Knochen spannt.

Die Einrichtung zur lösbaren Festlegung des Magazins an der Knochenplatte kann gemäß einer bevorzugten Ausführungsform elastische Rastglieder umfassen, die das Magazin mittels einer Schnappverbindung an der Knochenplatte halten. Es ist damit möglich, das Magazin auf eine Knochenplatte aufzuschnappen und auch in entsprechender Weise wieder zu lösen, ohne dass zusätzliche Werkzeuge oder Befestigungselemente notwendig wären.

So kann das Magazin an seiner Unterseite Rastelemente tragen, die bei auf die Knochenplatte aufgesetztem Magazin die Knochenplatte außenseitig umgreifen. Diese Konstruktion ermöglicht es, auch eine Festlegung von Magazinen an Knochenplatten vorzunehmen, die nicht speziell an die Geometrie der Magazine angepasst sind, es genügt, wenn die Rastelemente des Magazins die Außenkontur der Knochenplatte umgreifen.

Das Magazin kann insbesondere an seiner Unterseite eine Aufnahmevertiefung für die Aufnahme zumindest eines Teils der Knochenplatte aufweisen, deren Rand als elastisches Rastelement die in der Aufnahme aufgenommene Knochenplatte außenseitig umgreift.

Um eine sichere Positionierung der Knochenplatte relativ zum Magazin zu erreichen, können am Magazin und an der Knochenplatte Positionierungsvorsprünge und -rücksprünge angeordnet sein, die ineinandergreifen und dadurch das Magazin relativ zur Knochenplatte ausrichten.

Beispielsweise kann das Magazin an seiner Unterseite einen nach unten abstehenden Positionierungsstift tragen, der bei auf die Knochenplatte aufgesetztem Magazin in eine Positionierungsöffnung an der Knochenplatte eintaucht.

Günstig ist es, wenn am Magazin ein Handhabungsgriff festgelegt ist, dieser kann dann gleichzeitig zur Handhabung der gesamten Baueinheit aus Knochenplatte und Magazin dienen, wenn das Magazin an der Knochenplatte festgelegt ist. Bei einer bevorzugten Ausführungsform ist vorgesehen, dass das Magazin mehrere nebeneinander liegende Aufnahmekammern aufweist, in denen jeweils eine Knochenschraube derart angeordnet ist, dass sie bei auf die Knochenplatte aufgesetztem Magazin auf eine von mehreren Einschrauböffnungen der Knochenplatte gerichtet ist. Auf diese Weise können auch mehrere Knochenschrauben gleichzeitig relativ zu mehreren Einschrauböffnungen der Knochenplatte positioniert werden, und zwar mit einem einzigen Magazin, das auf der Knochenplatte lösbar festgelegt ist.

Bei einer besonders bevorzugten Ausführungsform kann vorgesehen sein, dass das Magazin eine Halterung umfasst, an der die Einrichtung zur lösbaren Fixierung an der Knochenplatte angeordnet ist, und ein an der Halterung verschieblich gelagertes Gehäuse, welches die Aufnahmekammer oder gegebenenfalls die mehreren Aufnahmekammern aufweist. Die Verschiebung kann dabei eine Translation oder eine Rotation sein oder auch eine aus Translation und Rotation überlagerte Bewegung. Durch die Verschieblichkeit des Gehäuses an der Halterung ist es möglich, die Aufnahmekammer oder die Aufnahmekammern in eine Position zu verschieben, in der sie mit einer bestimmten Einschrauböffnung ausgerichtet sind, in einer anderen Position sind sie dies nicht. Beispielsweise wäre es möglich, in verschiedenen Aufnahmekammern Knochenschrauben unterschiedlicher Größe aufzubewahren und durch Verschiebung des Gehäuses auszuwählen, welche der Knochenschrauben mit einer bestimmten Einschrauböffnung ausgerichtet wird.

Es ist auch möglich, dass das Gehäuse neben der Aufnahmekammer oder den Aufnahmekammern für Knochenschrauben mindestens eine weitere von der Oberseite zur Unterseite durchgehende Durchbrechung aufweist, die bei einer Verschiebung des Gehäuses mit einer oder mehreren Einschrauböffnungen der Knochenplatte ausrichtbar ist. Eine solche Durchbrechung kann beispielsweise als Bohrerführung ausgebildet sein und es dem Operateur ermöglichen, durch eine Einschrauböffnung hindurch eine Bohrung in dem unter der Knochenplatte angeordneten Knochen einzubringen, und zwar in Richtung der Durchbrechung. Durch die Durchbrechung hindurch könnte auch ein Markierungsvorgang erfolgen, beispielsweise mit Hilfe eines Körners, oder die Durchbrechung kann ein Werkzeug aufnehmen, mit dem ein Gewinde in den Knochen geschnitten wird.

Insbesondere kann vorgesehen sein, dass die Durchbrechung eine Aufnahmekammer für einen Dorn bildet, der in der Aufnahmekammer längsverschieblich gelagert ist. Ein solcher Dorn kann als Körner verwendet werden, wenn er in distaler Richtung gegen den Knochen verschoben wird und dann mit einer Spitze im Knochen eine Vertiefung bildet.

Günstig ist es, wenn der Dorn durch eine Feder in eine proximale Endstellung verschoben wird und entgegen der Wirkung dieser Feder in distaler Richtung verschiebbar ist.

Das Gehäuse kann an der Halterung längsverschieblich gelagert sein, bei einer besonders bevorzugten Ausführungsform ist vorgesehen, dass das Gehäuse an der Halterung verdrehbar gelagert ist derart, dass bei verschiedener Winkelstellung verschiedene Aufnahmekammern und Durchbrüche mit den Einschrauböffnungen ausrichtbar sind. Das Gehäuse ist damit an der Halterung nach Art eines Revolvers gelagert.

Es ist dabei vorteilhaft, wenn an dem Gehäuse ein Drehgriff angeordnet ist, mit dessen Hilfe die Verdrehung des Gehäuses relativ zur der Halterung erfolgen kann.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer Knochenplatte in Anlage an Wirbelkörpern mit zwei aufgesetzten Knochenschraubenmagazinen und einem Einschraubwerkzeug;
- Figur 2:: eine vergrößerte Detailansicht der Knochenplatte der Figur 1;
- Figur 3:: eine perspektivische Explosionsdarstellung der Knochenplatte der Figur 1 von der Unterseite der Knochenplatte her gesehen;
- Figur 4:: eine Schnittansicht längs Linie 4-4 in Figur 3;
- Figur 5:: eine Teilansicht eines abgewandelten Ausführungsbeispiels eines auf eine Knochenplatte aufgesetzten Magazins mit unmittelbar am Magazin angeordneten Rastvorsprüngen;
- Figur 6:: eine Schnittansicht eines Teils der Knochenplatte nach vollständigem Einschrauben der Knochenschraube und nach Abnahme des Magazin;
- Figur 7:: eine perspektivische Ansicht einer Knochenplatte mit zwei Magazinen mit verdrehbarem Gehäuse;
- Figur 8:: eine vergrößerte Ansicht der Knochenplatte der Figur 7 mit Magazinen zur Aufnahme von Knochenschrauben und verschiebbaren Dornen;
- Figur 9:: eine perspektivische Ansicht einer Knochenplatte mit einem darauf aufgesetzten Magazin mit verdrehbarem Gehäuse bei Ausrichtung der Einschrauböffnungen mit verschiebbaren Dornen;
- Figur 10:: eine perspektivische Ansicht der Knochenplatte und des Magazins der Figur 9 von der Unterseite her;
- Figur 11:: eine perspektivische Ansicht des Magazins der Figur 9 mit einem um 90° gedrehten Gehäuse und mit den Einschrauböffnungen ausgerichteten Knochenschrauben und
- Figur 12:: eine perspektivische Ansicht eines weiteren bevorzugten Ausführungsbeispiels eines Magazins ähnlich dem der Figur 9 in teilweise aufgeschnittener Darstellung mit einer Fixiereinrichtung für die Knochenschraube in Form eines Gewindes.

In der Zeichnung ist beispielhaft eine Knochenplatte 1 dargestellt, die in diesem Falle zwei Endstücke 2, 3 aufweist, die über zwei stabförmige Verbindungselemente 4, 5 miteinander verbunden sind. Die Endstücke 2, 3 sind plattenförmig ausgebildet und weisen jeweils zwei nebeneinander liegende Einschrauböffnungen 6 auf, die sich mit abgerundeten Seitenwänden 7 zur Unterseite hin verengen. Die Verbindungselemente 4, 5 können aus einem körperverträglichen Metall bestehen, beispielsweise aus Titan oder einer Titanlegierung, während die Endstücke 2, 3 aus einem körperverträglichen und biokompatiblen Kunststoff bestehen können, beispielsweise aus Polyetheretherketon (PEEK). Der Kunststoff kann gegebenenfalls auch durch Fasern verstärkt sein, beispielsweise durch Kohlefasern.

Auf diese Knochenplatte 1 können auf jedes der beiden Endstücke 2, 3 Magazine 8 aufgesetzt werden. Dabei sind die Magazine gleich aufgebaut, die wahlweise auf das eine Endstück oder das andere Endstück aufgesetzt werden können, es wird daher nachstehend nur eines der beiden Magazine 8 näher erläutert.

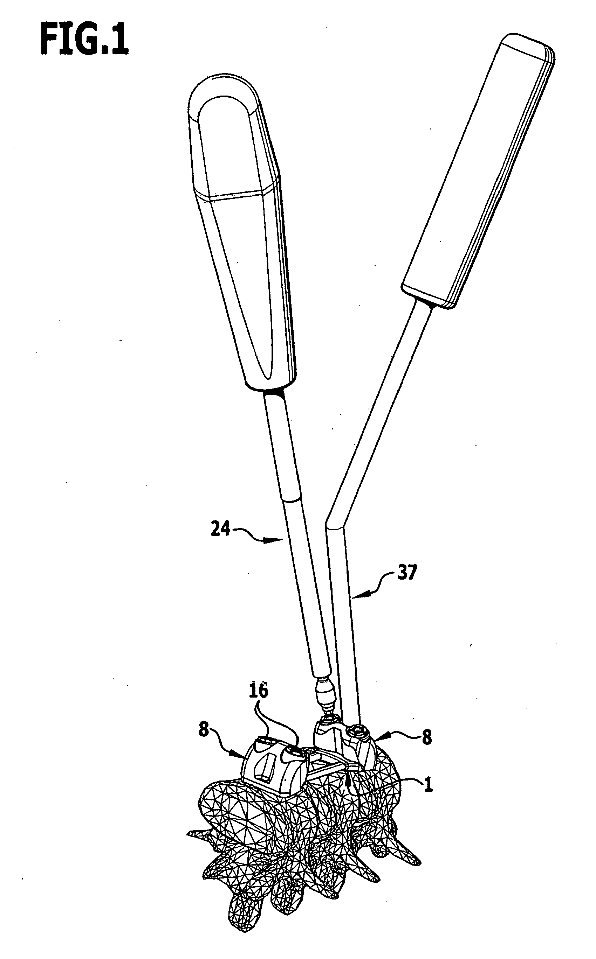

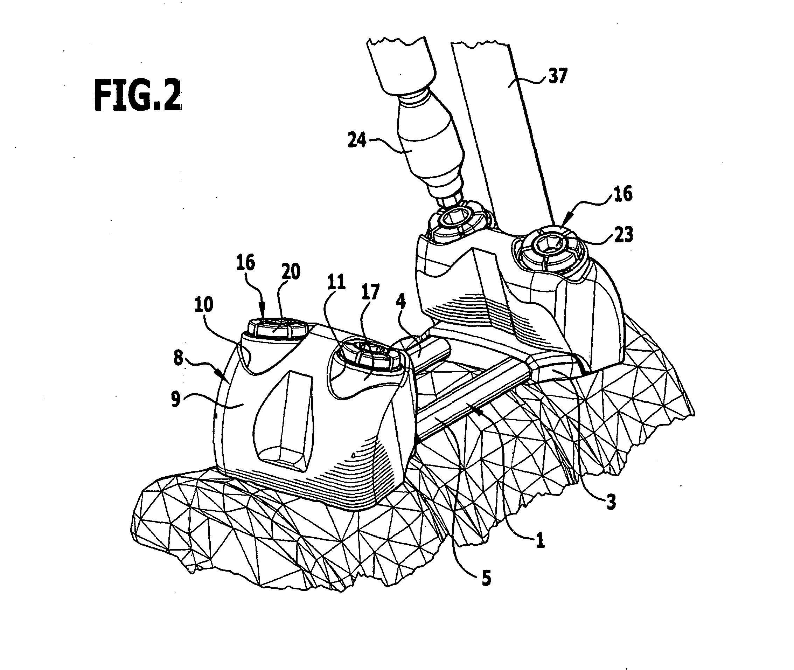

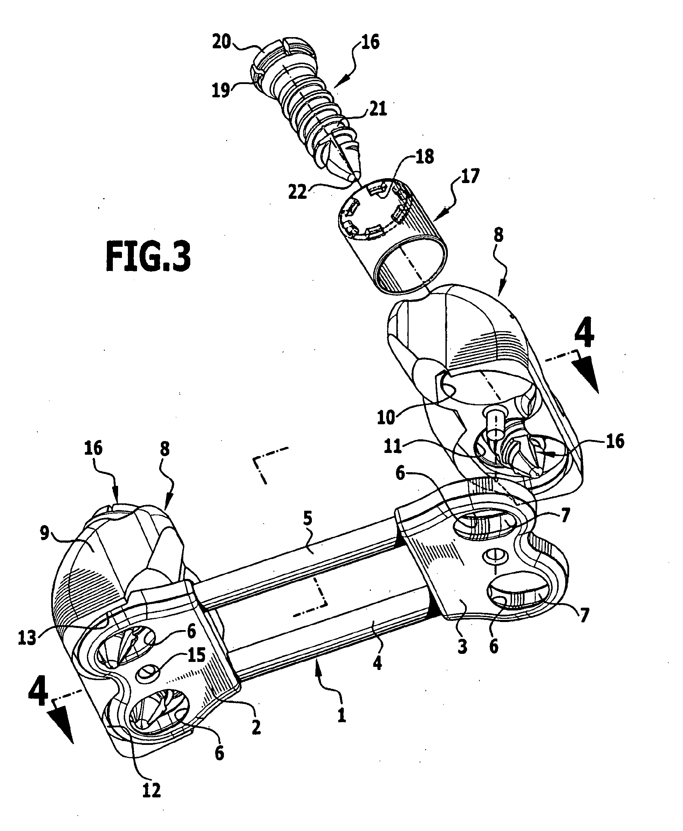

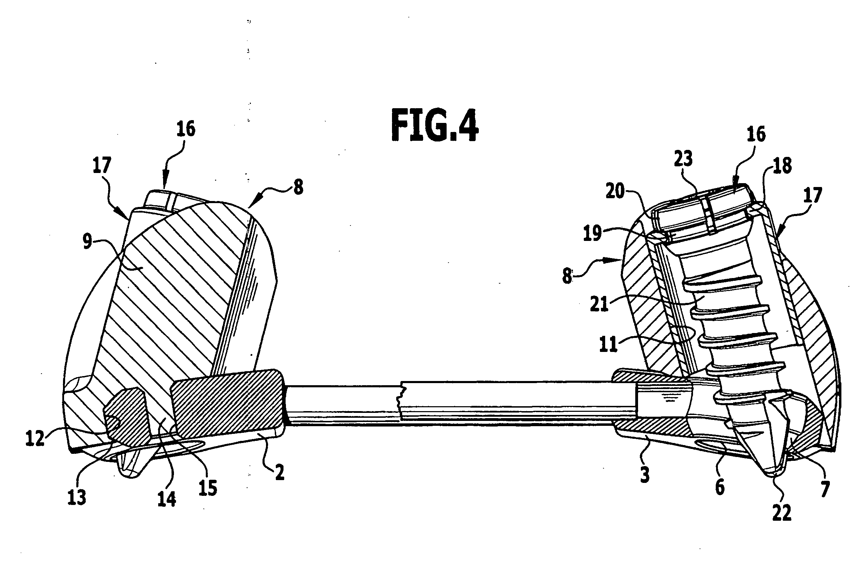

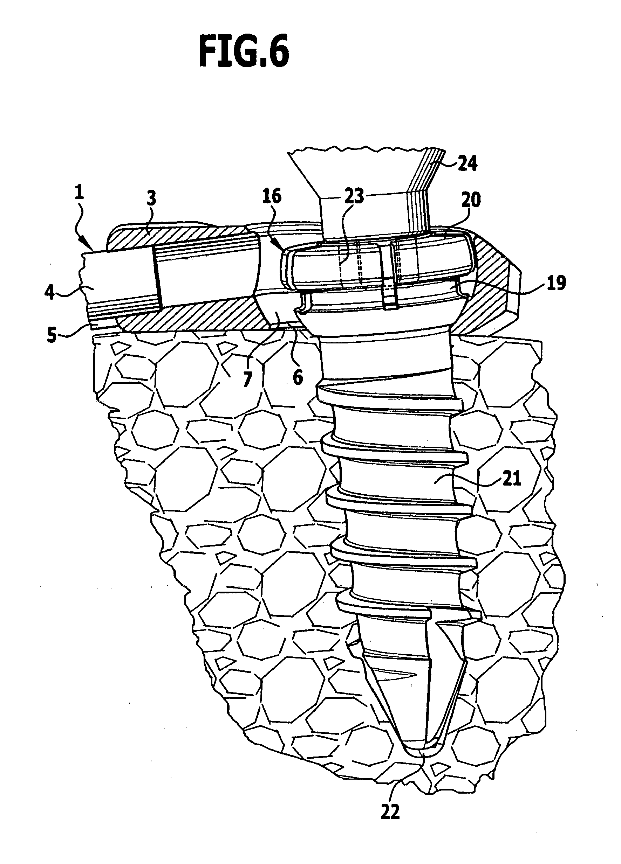

Das in den Figuren 1 bis 6 dargestellte Magazin 8 umfasst einen einteiligen Gehäuseblock 9 mit zwei nebeneinander angeordneten, kanalförmigen Aufnahmekammern 10, 11, die den Gehäuseblock.9 von der Oberseite zur Unterseite vollständig durchsetzen.

Der Gehäuseblock 9 ist lösbar auf jeweils eines der beiden Endstücke 2, 3 aufsetzbar, zur Festlegung des Gehäuseblockes 9 auf dem Endstück 2 oder 3 weist der Gehäuseblock 9 an seiner Unterseite eine an die Außenkontur des Endstückes angepasste Ausnehmung 12 auf, deren seitlicher Rand 13 geringfügig nach innen in die Ausnehmung 12 vorsteht. Der Gehäuseblock 9 ist aus einem sterilisierbaren Kunststoffmaterial gefertigt, beispielsweise aus Polyetheretherketon, Acrylbutadienstyrol, Polypropylen, Polyethylen, Polysulfon, Polyetherketon, Polyoxymethylen oder Polystyrol. Diese Materialien sind so elastisch, dass auch der Rand 13 elastisch nach außen verbiegbar ist. Dadurch kann der Gehäuseblock 9 auf ein Endstück 2 aufgedrückt werden, dabei gleitet der Rand 13 der Ausnehmung 12 an der Außenkontur des Endstückes 2 oder 3 entlang und umgreift dann das Endstück 2, 3 sobald dieses in die Ausnehmung 12 eintaucht. Damit erhält man eine elastische Rastverbindung oder Schnappverbindung, durch welche der Gehäuseblock 9 an dem Endstück 2 lösbar festgelegt ist. Zum Lösen genügt es, den Gehäuseblock 9 kräftig von dem Endstück 2 oder 3 abzuziehen, dabei wird der Rand 13 elastisch nach außen gebogen und gibt das Endstück 2 oder 3 frei.

Zur exakten Positionierung des Gehäuseblockes 9 trägt dieser an seiner Unterseite einen zapfenförmigen Vorsprung 14, der in eine Öffnung 15 des Endstückes 2 oder 3 eintaucht, diese Öffnung 15 ist zwischen den beiden Einschrauböffnungen 6 des Endstückes 2, 3 angeordnet.

Wenn der Gehäuseblock 9 in dieser Weise auf ein Endstück 2 aufgesetzt ist, sind die Aufnahmekammern 10, 11 des Gehäuseblockes 9 mit den beiden Einschrauböffnungen 6 ausgerichtet, so dass diese Aufnahmekammern 10, 11 einen von der Oberseite des Gehäuseblockes 9 auf die beiden Einschrauböffnungen 6 gerichteten Kanal ausbilden.

In diesen Aufnahmekammern 10, 11 ist bei dem Ausführungsbeispiel der Figuren 1 bis 6 jeweils eine Knochenschraube 16 aufgenommen, die in der Aufnahmekammer 10 zunächst in einer Anfangslage gehalten sind. Um die Knochenschraube 16 in dieser Anfangslage im Gehäuseblock 9 festzulegen, ist bei dem Ausführungsbeispiel der Figuren 1 bis 3 in jede Aufnahmekammer 10, 11 eine Haltehülse 17 eingesetzt, die in der Aufnahmekammer 10 entweder im Reibsitz oder in anderer Weise festgelegt werden kann, beispielsweise auch durch Verklebung oder durch eine Rastverbindung. Diese Haltehülse 17 trägt an ihrem oberen Rand nach innen vorstehende Rastvorsprünge 18, die in eine Umfangsnut 19 der Knochenschraube 16 eintauchen. Diese Umfangsnut 19 ist im Bereich des Kopfes 20 der Knochenschraube 16 angeordnet, der in der Anfangsstellung der Knochenschraube 16 damit am oberen Ende der Haltehülse 17 angeordnet ist und geringfügig nach oben über diese Haltehülse 17 vorsteht. Der sich an den Kopf 20 anschließende Gewindeschaft 21 der Knochenschraube 16 erstreckt sich dann über die Länge der Aufnahmekammer, die Spitze 22 der Knochenschraube 16 tritt dabei geringfügig in die Einschrauböffnung 6 ein, so dass die Spitze 22 nur ganz geringfügig nach unten über die Knochenplatte 1 vorsteht.

Im Kopf 20 der Knochenschraube 16 ist ein Innenmehrkant 23 angeordnet, dieser bildet eine Aufnahmeöffnung zum formschlüssigen Einführen eines Einschraubwerkzeuges 24.

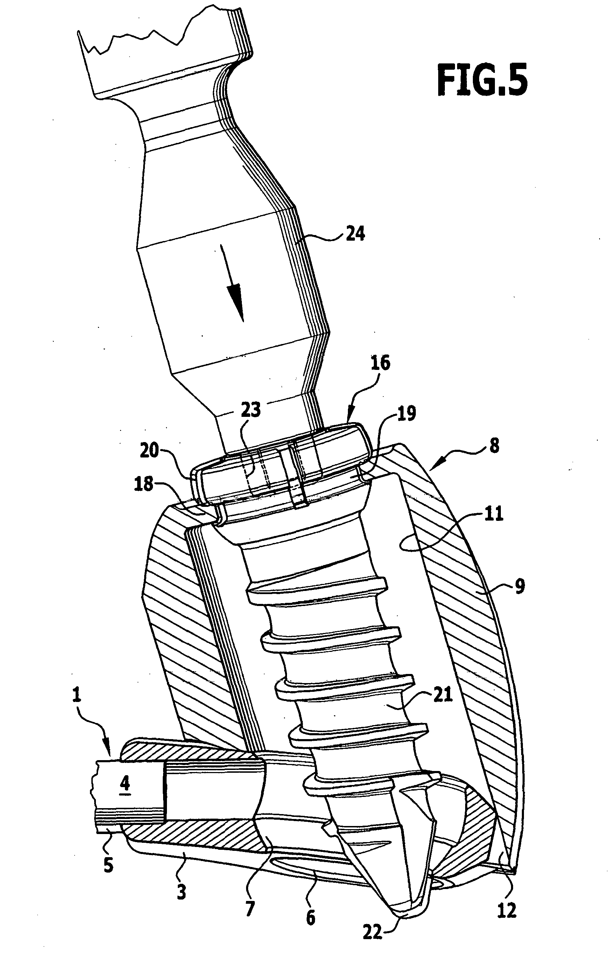

Bei dem Ausführungsbeispiel der Figur 5 ist eine ähnliche Ausgestaltung dargestellt, einander entsprechende Teile tragen daher dieselben Bezugszeichen. Im Unterscheid zu dem Ausführungsbeispiel der Figuren 1 bis 4 fehlt bei diesem Ausführungsbeispiel eine Haltehülse 17, bei diesem Ausführungsbeispiel sind die Rastvorsprünge 18 unmittelbar am Gehäuseblock 9 angeordnet.

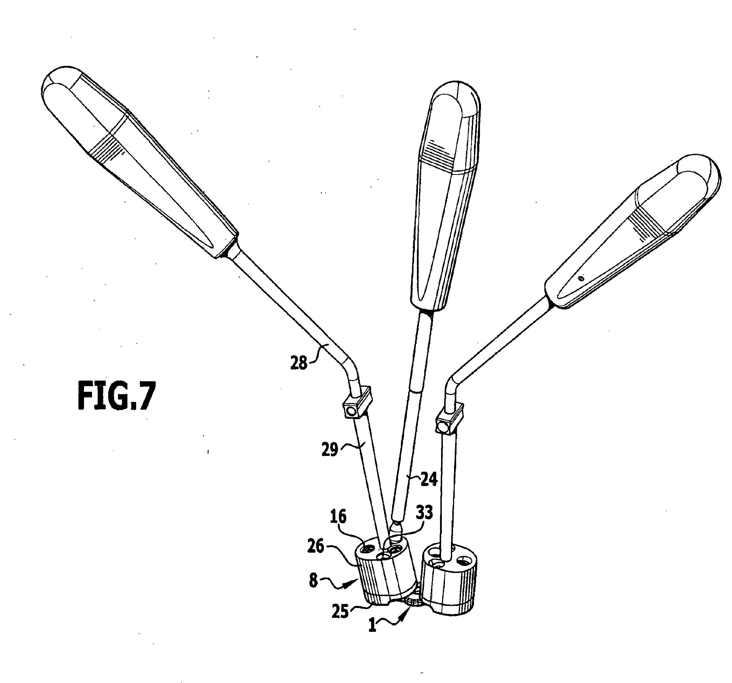

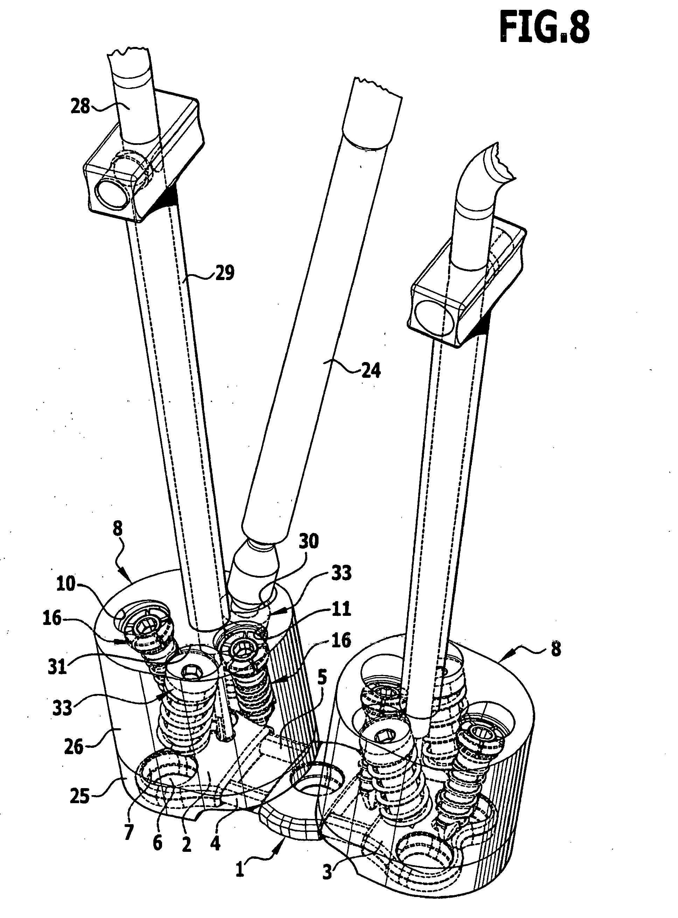

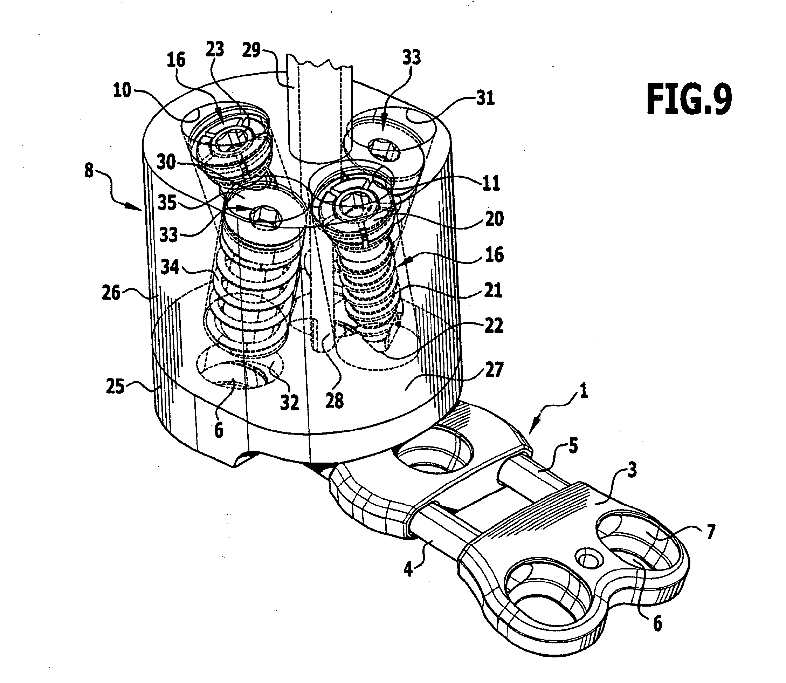

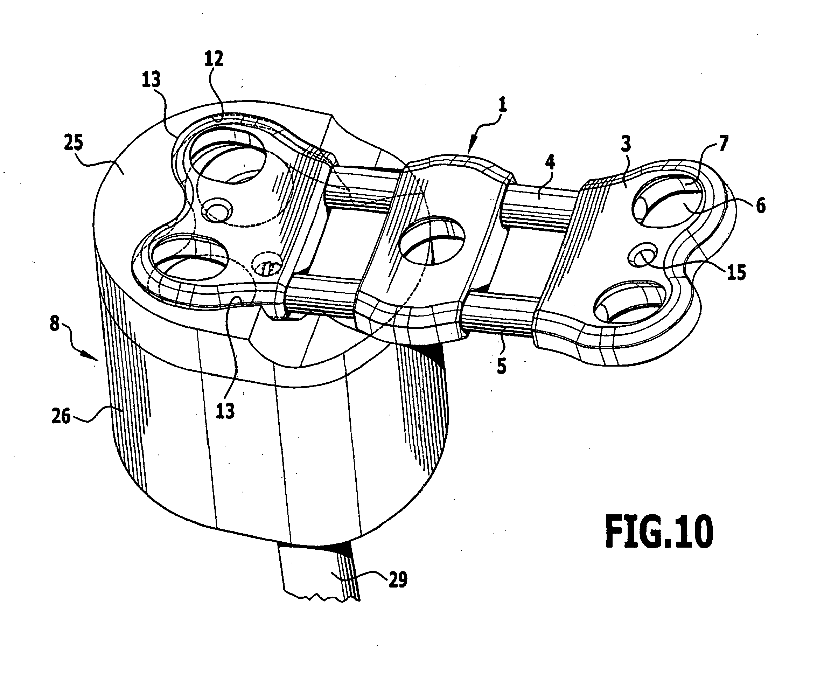

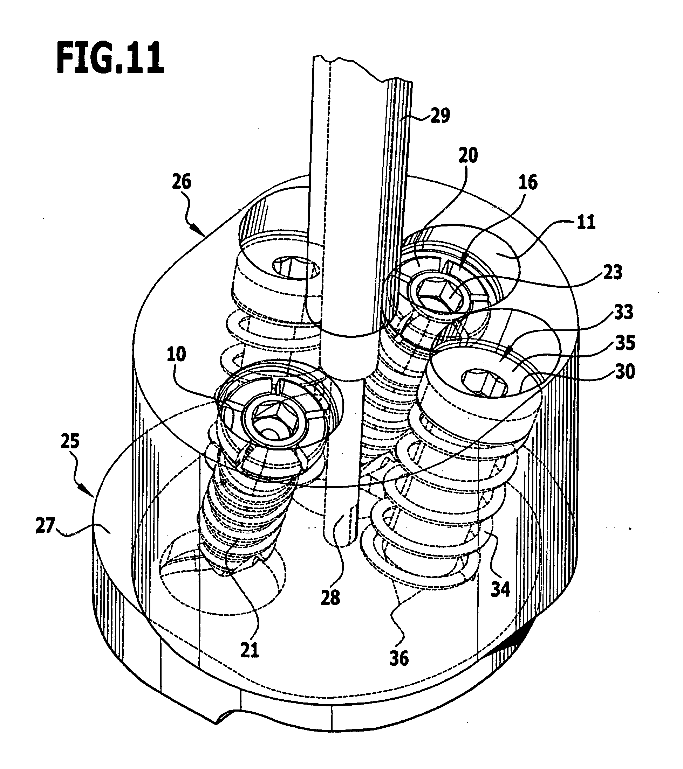

Bei dem in den Figuren 9 bis 11 dargestellten Ausführungsbeispiel des Magazins ist ebenfalls ein ähnlicher Aufbau gewählt, auch hier sind die einander entsprechenden Teile mit denselben Bezugszeichen gekennzeichnet. Im Unterschied zu den Ausführungsbeispielen der Figuren 1 bis 6 umfasst das Magazin 8 bei dem Ausführungsbeispiel der Figuren 7 bis 11 eine Halterung 25 und ein Gehäuse 26, die gemeinsam den Gehäuseblock 9 des Ausführungsbeispiels der Figuren 1 bis 6 ersetzen. Die Halterung 25 dient der Festlegung des Magazins an der Knochenplatte 1, sie ist an ihrer Unterseite genauso ausgebildet wie bei dem Ausführungsbeispiel der Figuren 1 bis 6 beschrieben, das heißt die Halterung 25 kann lösbar auf jeweils ein Endstück 2, 3 der Knochenplatte 1 aufgeschnappt werden. Die Halterung 25 hat die Form einer Platte mit einer ebenen Oberseite 27, und auf dieser plattenförmigen Halterung ist das Gehäuse 26 aufgesetzt, so dass das Gehäuse 26 mit seiner Unterseite auf der Oberseite 27 aufruht. Das Gehäuse 26 ist mit der Halterung 25 drehbar verbunden, die Drehachse verläuft senkrecht zur Oberseite 27 des Gehäuses 26 und liegt in der Mitte der Oberseite 27. Zur Lagerung des Gehäuses 26 an der Halterung 25 ist eine senkrechte Lagerwelle 28 vorgesehen, die nach oben aus dem Gehäuse 26 hervorsteht und die als Führung für ein Drehwerkzeug 29 dient, das von oben her über die Lagerwelle 28 geschoben werden kann. Das Drehwerkzeug greift in aus der Zeichnung nicht näher ersichtlicher Weise drehfest in das Gehäuse 26 ein, so dass beim Verdrehen des Drehwerkzeuges 29 das Gehäuse 26 relativ zur Halterung 25 verdreht wird.

Die Anordnung kann so gewählt sein, dass das Gehäuse 26 in axialer Richtung an der Halterung 25 festgelegt ist, und es kann auch vorgesehen sein, dass das Drehwerkzeug 29 in axialer Richtung fest mit dem Gehäuse 26 verbunden ist, so dass das Drehwerkzeug 29 dann auch gleichzeitig als Handhabungswerkzeug für das gesamte Magazin und auch für die Knochenplatte mit aufgesetztem Magazin dienen kann.

In dem Gehäuse 26 sind bei dem in den Figuren 7 bis 11 dargestellten Ausführungsbeispiel über den Umfang verteilt vier Aufnahmekammern 10, 11, 30, 31 angeordnet, die bei entsprechender Winkelstellung des Gehäuses 26 wahlweise mit den Einschrauböffnungen 6 der Knochenplatte 1 ausgerichtet sind oder gegenüber diesen versetzt sind. Dabei ist vorgesehen, dass die Halterung 25 oberhalb der Einschrauböffnungen 6 angeordnete, fensterartige Durchbrechungen 32 aufweist, so dass aus den mit den Einschrauböffnungen ausgerichteten Aufnahmekammern 10, 11 oder 30, 31 ein direkter Zugang zu den entsprechenden Einschrauböffnungen 6 gegeben ist.

Wie bei dem Ausführungsbeispiel der Figuren 1 bis 6 sind in den Aufnahmekammern 10, 11 Knochenschrauben 16 in ihrer Anfangsstellung gehalten, die in gleicher Weise bei Ausrichtung mit den Einschrauböffnungen 6 durch diese vorgeschoben und dann in den darunterliegenden Knochen eingeschraubt werden können.

In den beiden anderen Aufnahmekammern 30, 31 sind bei den Ausführungsbeispielen der Figuren 7 bis 11 längsverschieblich dornförmige Stifte 33 gelagert, die von einer Schraubenfeder 34 umgeben werden. Diese Stifte werden durch die Schraubenfeder 34, die sich an einer kopfförmigen Verbreiterung 35 der Stifte 33 abstützt, in eine proximale Ausgangsstellung verschoben und können gegen die Kraft der Schraubenfeder 34 in distaler Richtung in der Aufnahmekammer 30, 31 verschoben werden, so dass sie bei Ausrichtung dieser Aufnahmekammer 30, 31 durch die Einschrauböffnung 6 hindurchragen und dort mit einer Spitze 36 eine Vertiefung in dem unter der Knochenplatte 1 liegenden Knochen erzeugen, es handelt sich somit um ein Werkzeug zum Ankörnen des Knochens.

Grundsätzlich wäre es auch möglich, in einer der Aufnahmekammern 30, 31 weder einen Dorn noch eine Knochenschraube anzuordnen, sondern diese leer zu lassen und die entsprechende Durchbrechung dann als Führung für ein Bohrwerkzeug, ein Gewindeschneidwerkzeug oder ähnliches einzusetzen. Wesentlich ist lediglich die Ausrichtung der entsprechenden Aufnahmekammer 30, 31 mit einer Einschrauböffnung 6, wenn das Gehäuse 26 in einer entsprechenden Winkellage steht.

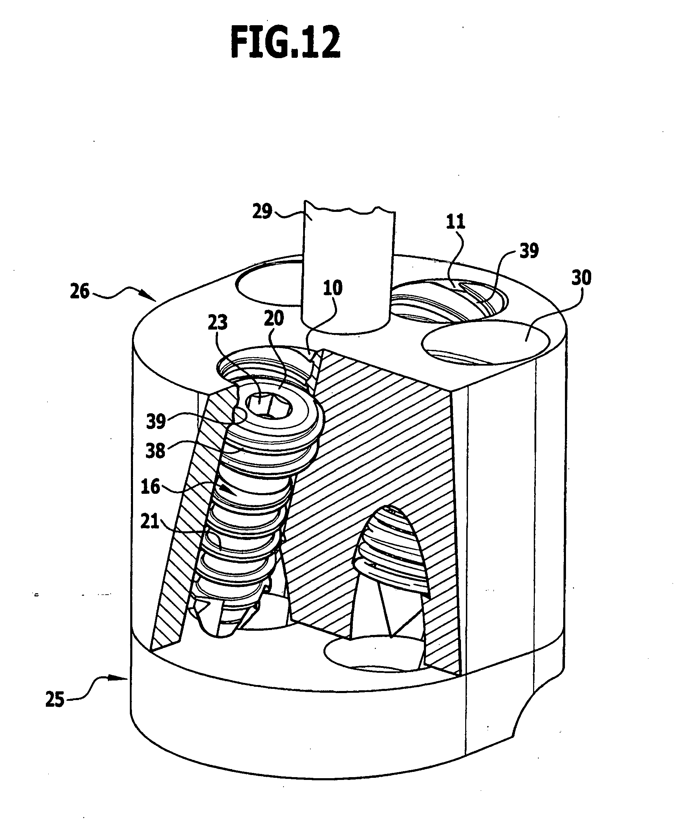

Bei den vorstehend beschriebenen Ausführungsbeispielen erfolgt die Festlegung der Knochenschrauben in dem Gehäuseblock durch Rastvorsprünge 18, die in die Umfangsnut 19 der Knochenschraube 16 eintauchen. Bei dem Ausführungsbeispiel der Figur 12, bei dem ein ähnlicher Aufbau gewählt ist wie bei den Ausführungsbeispielen der Figuren 9 bis 11 und bei dem entsprechende Teile dieselben Bezugszeichen tragen, ist die Außenseite des Kopfes 20 der Knochenschraube 16 mit einem Außengewinde 38 versehen, dessen Steigung dem Gewinde des Gewindeschaftes 21 entspricht. In die Innenwand der Aufnahmekammern 10, 11 ist an der Oberseite des Gehäuseblockes 9 ein entsprechendes Innengewinde 39 eingearbeitet, in welches das Außengewinde 38 des Kopfes 20 eingeschraubt ist, so dass dadurch eine Fixierung der Knochenschraube in der Aufnahmekammer erreicht wird. Diese Fixierung wird durch das Einschrauben gelöst, da das Außengewinde 38 des Kopfes 20 nach einigen Umdrehungen aus dem Innengewinde 39 austritt und dann in einem erweiterten Bereich der Aufnahmekammer 10, 11 ohne Eingriff in Richtung auf den Knochen verschoben werden kann. Die Gänge des Innengewindes 39 stehen also als Vorsprünge in den Querschnitt der Aufnahmekammer 10, 11 vor. Eine solche Ausgestaltung kann selbstverständlich auch bei den Ausführungsbeispielen der Figuren 1 bis 6 Verwendung finden.

Wenn bei einer weiteren bevorzugten Ausgestaltung der gewindeförmige Vor- oder Rücksprung Teil des Gewindes des Gewindeschaftes ist, kann beispielsweise vorgesehen sein, dass der Kerndurchmesser im Gewindeschaft kleiner ist als im Bereich des Vor- oder Rücksprunges, so dass die Knochenschraube insgesamt im Kopfbereich einen verdickten Kern aufweist und somit weiterhin geeignet ist, eine Knochenplatte gegen einen Knochen zu drücken, wenn die Knochenschraube in den Knochen eingeschraubt ist. Der Außendurchmesser der Gewindegänge ist aber im Bereich des Gewindeschaftes und im Bereich der Vor- oder Rücksprünge am oberen Ende der Knochenschraube gleich, das heißt, die Knochenschraube weist in diesem Falle ein durchgehendes Gewinde a uf.

Das Magazin 8 der vorstehend beschriebenen Ausgestaltungen kann für sich genommen als vorkonfektionierte, sterile Baueinheit geliefert werden, in der Knochenschrauben und gegebenenfalls dornförmige Stifte bereits eingesetzt sind und dort in der Ausgangslage stehen. Grundsätzlich wäre es auch möglich, bereits Knochenplatten mit entsprechend aufgesetzten Magazinen als Baueinheit in steriler Verpackung zu liefern, so dass der Benutzer dann lediglich eine derartige Baueinheit in der gewünschten Weise am Knochen platzieren muss und dann sofort die Möglichkeit hat, die Knochenschrauben durch die entsprechenden Einschrauböffnungen 6 hindurchzudrücken und einzuschrauben. Ein Einsetzen der Knochenschrauben und eine Führung der Knochenschrauben müssen nicht mehr erfolgen, diese Aufgaben werden von dem Magazin übernommen.

## Patentansprüche

1. Knochenplatte (1) mit einem lösbar darauf festgelegten Magazin (8) zur Aufnahme von mindestens einer Knochenschraube (16) mit mindestens einer von der Oberseite zur Unterseite des Magazins (8) durchgehenden Aufnahmekammer (10, 11), in der eine Knochenschraube (16) derart angeordnet ist, dass ihre distale Spitze (22) auf eine Einschrauböffnung (6) der Knochenplatte (1) gerichtet ist und ihr proximales Ende von der Oberseite des Magazins (8) für ein Einschraubwerkzeug (24) zugänglich ist, und mit einer Fixiervorrichtung (18, 19) zur lösbaren Festlegung der Knochenschraube (16) in der Aufnahmekammer (10, 11).

2. Knochenplatte nach Anspruch 1, **dadurch gekennzeichnet, dass** die Aufnahmekammer (10, 11) die Form eines Kanals aufweist.

3. Knochenplatte nach einem der Ansprüche 1 oder 2, **dadurch gekennzeichnet, dass** die Aufnahmekammer (10, 11) eine Längsführung für die Knochenschraube (16) bildet, längs welcher die Knochenschraube (16) beim Einschrauben in distaler Richtung durch die Einschrauböffnung (6) hindurch vorschiebbar ist.

4. Knochenplatte nach einem der voranstehenden Ansprüche, **dadurch gekennzeichnet, dass** die Knochenschraube (16) im Reibsitz an der Innenwand der Aufnahmekammer (10, 11) anliegt und dadurch lösbar in der Aufnahmekammer (10, 11) gehalten ist.

5. Knochenplatte nach einem der Ansprüche 1 bis 3, **dadurch gekennzeichnet, dass** die Fixiervorrichtung mindestens einen elastischen Rastvorsprung (18) und mindestens einen mit diesem zusammenwirkenden Rastrücksprung (19) umfasst.

6. Knochenplatte nach Anspruch 5, **dadurch gekennzeichnet, dass** mindestens ein elastischer Rastvorsprung (18) am Magazin (8) in die Aufnahmekammer (10, 11) hineinragt und in einen Rücksprung (19) der Knochenschraube (16) eintaucht.

7. Knochenplatte nach Anspruch 6, **dadurch gekennzeichnet, dass** der elastische Rastvorsprung (18) an einem in die Aufnahmekammer (10, 11) eingesetzten Halteelement (17) angeordnet ist.

8. Knochenplatte nach einem der Ansprüche 6 oder 7, **dadurch gekennzeichnet, dass** der Rastvorsprung (18) am Magazin (8) in einen Rastrücksprung (19) in Form einer an der Knochenschraube (16) vorgesehenen Umfangsnut eintaucht.

9. Knochenplatte nach einem der Ansprüche 1 bis 3, **dadurch gekennzeichnet, dass** die Fixiereinrichtung einen Vorsprung (38) an der Knochenschraube (16) oder der Aufnahmekammer (10, 11) und einen Rücksprung (39) an der Aufnahmekammer (10, 11) beziehungsweise der Knochenschraube (16) aufweist, in den der Vorsprung (38) eingreift, und dass der Vorsprung (38) und/oder der Rücksprung (39) nach Art eines Gewindes wendelförmig ausgebildet sind.

10. Knochenplatte nach Anspruch 9, **dadurch gekennzeichnet, dass** die Steigung des wendelförmigen Vorsprunges (38) und/oder Rücksprungs (39) der Steigung des Gewindes der Knochenschraube (16) entspricht.

11. Knochenplatte nach Anspruch 10, **dadurch gekennzeichnet, dass** der Vorsprung (38) oder Rücksprung (39) an der Knochenschraube (16) zumindest teilweise von dem Gewinde der Knochenschraube (16) gebildet werden.

12. Knochenplatte nach einem der voranstehenden Ansprüche, **dadurch gekennzeichnet, dass** die Einrichtung zur lösbaren Festlegung des Magazins (8) an der Knochenplatte (1) elastische Rastglieder (13) umfasst, die das Magazin (8) mittels einer Schnappverbindung an der Knochenplatte (1) halten.

13. Knochenplatte nach Anspruch 12, **dadurch gekennzeichnet, dass** das Magazin (8) an seiner Unterseite Rastelemente (13) trägt, die bei auf die Knochenplatte (1) aufgesetztem Magazin (8) die Knochenplatte (1) außenseitig umgreifen.

14. Knochenplatte nach Anspruch 13, **dadurch gekennzeichnet, dass** das Magazin (8) an seiner Unterseite eine Aufnahmevertiefung (12) für die Aufnahme zumindest eines Teils der Knochenplatte (1) aufweist, deren Rand (13) als elastisches Rastelement die in der Aufnahmevertiefung (12) aufgenommene Knochenplatte (1) außenseitig umgreift.

15. Knochenplatte nach einem der voranstehenden Ansprüche, **dadurch gekennzeichnet, dass** am Magazin (8) und an der Knochenplatte (1) Positioniervorsprünge und -rücksprünge (14, 15) angeordnet sind, die ineinander greifen und dadurch das Magazin (8) relativ zur Knochenplatte (1) ausrichten.

16. Knochenplatte nach Anspruch 15, **dadurch gekennzeichnet, dass** das Magazin (8) an seiner Unterseite einen nach unten abstehenden Positionierungsstift (14) trägt, der bei auf die Knochenplatte (1) aufgesetztem Magazin (8) in eine Positionierungsöffnung (15) der Knochenplatte (1) eintaucht.

17. Knochenplatte nach einem der voranstehenden Ansprüche, **dadurch gekennzeichnet, dass** am Magazin (8) ein Handhabungsgriff (37) festgelegt ist.

18. Knochenplatte nach einem der voranstehenden Ansprüche, **dadurch gekennzeichnet, dass** das Magazin (8) mehrere nebeneinander liegende Aufnahmekammern (10, 11) aufweist, in denen jeweils eine Knochenschraube (16) derart angeordnet ist, dass sie bei auf die Knochenplatte (1) aufgesetztem Magazin (8) auf eine von mehreren Einschrauböffnungen (6) der Knochenplatte (1) gerichtet ist.

19. Knochenplatte nach einem der voranstehenden Ansprüche, **dadurch gekennzeichnet, dass** das Magazin (8) eine Halterung (25) umfasst, an der die Einrichtung (12, 13) zur lösbaren Fixierung an der Knochenplatte (1) angeordnet ist, und ein an der Halterung (25) verschieblich gelagertes Gehäuse (26), welches die Aufnahmekammer (10, 11) oder gegebenenfalls die mehreren Aufnahmekammern aufweist.

20. Knochenplatte nach Anspruch 19, **dadurch gekennzeichnet, dass** das Gehäuse (26) neben der Aufnahmekammer oder den Aufnahmekammern (10, 11) für Knochenschrauben (16) mindestens eine weitere von der Oberseite zur Unterseite durchgehende Durchbrechung (30, 31) aufweist, die bei einer Verschiebung des Gehäuses (26) mit einer oder mehreren Einschrauböffnungen (6) der Knochenplatte (1) ausrichtbar ist.

21. Knochenplatte nach Anspruch 20, **dadurch gekennzeichnet, dass** die Durchbrechung (30, 31) eine Aufnahmekammer für einen Dorn (33) bildet, der in der Aufnahmekammer längsverschieblich gelagert ist.

22. Knochenplatte nach Anspruch 21, **dadurch gekennzeichnet, dass** der Dorn (33) durch eine Feder (34) in eine proximale Endstellung verschoben wird und entgegen der Wirkung dieser Feder (34) in distaler Richtung verschiebbar ist.

23. Knochenplatte nach einem der Ansprüche 20 bis 22, **dadurch gekennzeichnet, dass** das Gehäuse (26) an der Halterung (25) verdrehbar gelagert ist, derart, dass bei verschiedener Winkelstellung verschiedene Aufnahmekammern (10, 11) und Durchbrüche (30, 31) mit den Einschrauböffnungen (6) ausrichtbar sind.

24. Knochenplatte nach Anspruch 23, **dadurch gekennzeichnet, dass** an dem Gehäuse (26) ein Drehgriff (29) angeordnet ist.

## Claims

1. Bone plate (1) comprising a releasably secured magazine (8) thereon for receiving at least one bone screw (16) and having a receiving chamber (10, 11) extending continuously from the upper side to the underside of the magazine (8), a bone screw (16) being arranged in the receiving chamber (8) in such a way that the distal tip (22) of the bone screw (16) is directed at a screw-in opening (6) in the bone plate (1), and the proximal end of the bone screw (16) is accessible to a screwing-in tool (24) from the upper side of the magazine (8), and a fixing device (18, 19) for releasably fixing the bone screw (16) in the receiving chamber (10, 11).

2. Bone plate in accordance with claim 1, **characterized in that** the receiving chamber (10, 11) is in the form of a channel.

3. Bone plate in accordance with claim 1 or 2, **characterized in that** the receiving chamber (10, 11) forms a longitudinal guide for the bone screw (16), along which the bone screw (16) is advanceable, while being screwed in, in the distal direction through the screw-in opening (6).

4. Bone plate in accordance with any one of the preceding claims, **characterized in that** the bone screw (16) abuts with a frictional fit on the inside wall of the receiving chamber (10, 11) and is thereby releasably held in the receiving chamber (10, 11).

5. Bone plate in accordance with any one of claims 1 to 3, **characterized in that** the fixing device comprises at least one elastic latching projection (18) and at least one latching recess (19) cooperating with the latching projection (18).

6. Bone plate in accordance with claim 5, **characterized in that** at least one elastic latching projection (18) on the magazine (8) extends into the receiving chamber (10, 11) and enters a recess (19) on the bone screw (16).

7. Bone plate in accordance with claim 6, **characterized in that** the elastic latching projection (18) is arranged on a retaining element (17) inserted into the receiving chamber (10, 11).

8. Bone plate in accordance with claim 6 or 7, **characterized in that** the latching projection (18) on the magazine (8) enters a latching recess (19) in the form of a circumferential groove provided on the bone screw (16).

9. Bone plate in accordance with any one of claims 1 to 3, **characterized in that** the fixing device comprises a projection (38) on the bone screw (16) or the receiving chamber (10, 11), and a recess (39) on the receiving chamber (10, 11) or the bone screw (16) for engagement of the projection (38) in the recess (39), and **in that** the projection (38) and/or the recess (39) is helically constructed in the manner of a thread.

10. Bone plate in accordance with claim 9, **characterized in that** the pitch of the at least one of the helical projection (38) and the helical recess (39) corresponds to the pitch of the thread of the bone screw (16).

11. Bone plate in accordance with claim 10, **characterized in that** the projection (38) or recess (39) on the bone screw (16) is formed, at least in part, by the thread of the bone screw (16).

12. Bone plate in accordance with any one of the preceding claims, **characterized in that** the device for the releasable securing of the magazine (8) to the bone plate (1) comprises elastic latching elements (13) which hold the magazine (8) by means of a snap-in connection on the bone plate (1).

13. Bone plate in accordance with claim 12, **characterized in that** the magazine (8) carries on its underside latching elements (13) which, when the magazine (8) is mounted on the bone plate (1), engage around the outer side of the bone plate (1).

14. Bone plate in accordance with claim 13, **characterized in that** the magazine (8) has on its underside a receiving recess (12) for receiving at least part of the bone plate (1), and the rim (13) of the receiving recess (12), as elastic latching element, engages around the outer side of the bone plate (1) located in the receiving recess (12).

15. Bone plate in accordance with any one of the preceding claims, **characterized in that** positioning projections (14) and recesses (15) are arranged on the magazine (8) and on the bone plate (1), the positioning projections (14) and recesses (15) engaging with one another and thereby aligning the magazine (8) relative to the bone plate (1).

16. Bone plate in accordance with claim 15, **characterized in that** the magazine (8) carries on its underside a downwardly projecting positioning pin (14) which enters a positioning opening (15) on the bone plate (1) when the magazine (8) is mounted on the bone plate (1).

17. Bone plate in accordance with any one of the preceding claims, **characterized in that** a handling grip (37) is secured to the magazine (8).

18. Bone plate in accordance with any one of the preceding claims, **characterized in that** the magazine (8) comprises several adjacent receiving chambers (10, 11), in each of which a bone screw (16) is arranged in such a way that when the magazine (8) is mounted on the bone plate (1), the bone screw (16) is directed at one of several screw-in openings (6) in the bone plate (1).

19. Bone plate in accordance with any one of the preceding claims, **characterized in that** the magazine (8) comprises a holder (25) on which the device (12, 13) for removable fixation to the bone plate (1) is arranged, and a housing (26) which is displaceably mounted on the holder (25) and comprises the receiving chamber (10, 11) or optionally the several receiving chambers.

20. Bone plate in accordance with claim 19, **characterized in that** the housing (26) comprises in addition to the receiving chamber or the receiving chambers (10, 11) for bone screws (16) at least one further through-opening (30, 31) which extends continuously from the upper side to the underside and, upon displacement of the housing (26), is alignable with one or more screw-in openings (6) in the bone plate (1).

21. Bone plate in accordance with claim 20, **characterized in that** the through-opening (30, 31) forms a receiving chamber for a spike-shaped pin (33) which is mounted for longitudinal displacement in the receiving chamber.

22. Bone plate in accordance with claim 21, **characterized in that** the spike-shaped pin (33) is displaced into a proximal end position by a spring (34) and is displaceable in the distal direction against the action of this spring (34).

23. Bone plate in accordance with any one of claims 20 to 22, **characterized in that** the housing (26) is mounted for rotation on the holder (25) in such a way that in different angular positions different receiving chambers (10, 11) and through-openings (30, 31) are alignable with the screw-in openings (6).

24. Bone plate in accordance with claim 23, **characterized in that** a rotary handle (29) is arranged on the housing (26).

## Revendications

1. Plaque d'ostéosynthèse (1) sur laquelle est fixé de manière amovible, un magasin (8), qui est destiné à recevoir au moins une vis à os ou vis d'ostéosynthèse (16), et présente au moins un compartiment de réception (10, 11) traversant du côté supérieur au côté inférieur du magasin (8), et dans lequel est agencée une vis d'ostéosynthèse (16) de manière telle, que sa pointe distale (22) soit dirigée vers une ouverture de vissage (6) de la plaque d'ostéosynthèse (1), et que son extrémité proximale soit accessible à partir du côté supérieur du magasin (8), pour un outil de vissage (24), et l'ensemble comprenant en outre un dispositif de fixation (18, 19) pour la fixation amovible de la vis d'ostéosynthèse (16) dans le compartiment de réception (10, 11).

2. Plaque d'ostéosynthèse selon la revendication 1, **caractérisée en ce que** le compartiment de réception (10, 11) présente la forme d'un canal.

3. Plaque d'ostéosynthèse selon l'une des revendications 1 ou 2, **caractérisée en ce que** le compartiment de réception (10, 11) forme un guide longitudinal pour la vis d'ostéosynthèse (16), le long duquel la vis d'ostéosynthèse (16) peut avancer en direction distale à travers l'ouverture de vissage (6), lors du vissage.

4. Plaque d'ostéosynthèse selon l'une des revendications précédentes, **caractérisée en ce que** la vis d'ostéosynthèse (16) s'appuie, selon un ajustement à friction, contre la paroi intérieure du compartiment de réception (10, 11), en étant ainsi maintenue de manière amovible dans le compartiment de réception (10, 11).

5. Plaque d'ostéosynthèse selon l'une des revendications 1 à 3, **caractérisée en ce que** le dispositif de fixation comprend au moins une protubérance d'encliquetage élastique (18) et au moins un retrait d'encliquetage (19) coopérant avec celle-ci.

6. Plaque d'ostéosynthèse selon la revendication 5, **caractérisée en ce qu'**au moins une protubérance d'encliquetage élastique (18) sur le magasin (8) s'engage à l'intérieur du compartiment de réception (10, 11), et vient s'insérer dans un retrait (19) de la vis d'ostéosynthèse (16).

7. Plaque d'ostéosynthèse selon la revendication 6, **caractérisée en ce que** la protubérance d'encliquetage élastique (18) est agencée sur un élément de support (17) inséré dans le compartiment de réception (10, 11).

8. Plaque d'ostéosynthèse selon l'une des revendications 6 ou 7, **caractérisée en ce que** la protubérance d'encliquetage (18) sur le magasin (8) vient s'insérer dans un retrait d'encliquetage (19) sous la forme d'une rainure périphérique prévue sur la vis d'ostéosynthèse (16).

9. Plaque d'ostéosynthèse selon l'une des revendications 1 à 3, **caractérisée en ce que** le dispositif de fixation présente une protubérance (38) sur la vis d'ostéosynthèse (16) ou le compartiment de réception (10, 11), et un retrait (39) sur le compartiment de réception (10, 11) ou respectivement la vis d'ostéosynthèse (16), dans lequel s'engage la protubérance (38), et **en ce que** la protubérance (38) et/ou le retrait (39) sont réalisés en forme d'hélice, à la manière d'un filetage.

10. Plaque d'ostéosynthèse selon la revendication 9, **caractérisée en ce que** le pas de la protubérance (38) et/ou du retrait (39) en forme d'hélice correspond au pas du filetage de la vis d'ostéosynthèse (16).

11. Plaque d'ostéosynthèse selon la revendication 10, **caractérisée en ce que** la protubérance (38) ou le retrait (39) sur la vis d'ostéosynthèse (16) sont formés au moins partiellement par le filetage de la vis d'ostéosynthèse (16).

12. Plaque d'ostéosynthèse selon l'une des revendications précédentes, **caractérisée en ce que** le dispositif pour la fixation amovible du magasin (8) sur la plaque d'ostéosynthèse (1), comprend des organes d'encliquetage élastiques (13), qui maintiennent le magasin (8) sur la plaque d'ostéosynthèse (1) au moyen d'une liaison par enclenchement.

13. Plaque d'ostéosynthèse selon la revendication 12, **caractérisée en ce que** le magasin (8) porte sur son côté inférieur, des éléments d'encliquetage (13) qui, lorsque le magasin (8) est rapporté sur la plaque d'ostéosynthèse (1), enserrent la plaque d'ostéosynthèse (1) par le côté extérieur.

14. Plaque d'ostéosynthèse selon la revendication 13, **caractérisée en ce que** le magasin (8) présente sur son côté inférieur, un creux de réception (12) destiné à recevoir au moins une partie de la plaque d'ostéosynthèse (1), et dont le bord (13), en guise d'élément d'encliquetage élastique, enserre par le côté extérieur, la plaque d'ostéosynthèse (1) reçue dans le creux de réception (12).

15. Plaque d'ostéosynthèse selon l'une des revendications précédentes, **caractérisée en ce que** sur le magasin (8) et sur la plaque d'ostéosynthèse (1) sont agencés des protubérances et des retraits de positionnement (14, 15), qui s'engagent mutuellement les uns dans les autres, en orientant ainsi le magasin (8) par rapport à la plaque d'ostéosynthèse (1).

16. Plaque d'ostéosynthèse selon la revendication 15, **caractérisée en ce que** le magasin (8) porte sur son côté inférieur, un tenon de positionnement (14) faisant saillie vers le bas, qui, lorsque le magasin (8) est rapporté sur la plaque d'ostéosynthèse (1), s'engage dans une ouverture de positionnement (15) de la plaque d'ostéosynthèse (1).

17. Plaque d'ostéosynthèse selon l'une des revendications précédentes, **caractérisée en ce que** sur le magasin (8) est fixée une poignée de manipulation (37).

18. Plaque d'ostéosynthèse selon l'une des revendications précédentes, **caractérisée en ce que** le magasin (8) présente plusieurs compartiments de réception (10, 11) placés côte à côte, dans chacun desquels est agencée respectivement une vis d'ostéosynthèse (16), de manière telle qu'elle soit dirigée, lorsque le magasin (8) est rapporté sur la plaque d'ostéosynthèse (1), sur l'une de plusieurs ouvertures de vissage (6) de la plaque d'ostéosynthèse (1).

19. Plaque d'ostéosynthèse selon l'une des revendications précédentes, **caractérisée en ce que** le magasin (8) comprend un support (25) sur lequel est agencé le dispositif (12, 13) pour la fixation amovible sur la plaque d'ostéosynthèse (1), et un boitier (26) qui est monté coulissant sur le support (25) et présente le compartiment de réception (10, 11) ou, le cas échéant, les plusieurs compartiments de réception.

20. Plaque d'ostéosynthèse selon la revendication 19, **caractérisée en ce que** le boitier (26), en-dehors du compartiment de réception ou des compartiments de réception (10, 11) pour des vis d'ostéosynthèse (16), présente au moins un autre évidement de passage (30, 31) traversant du côté supérieur au côté inférieur, qui, lors d'un coulissement du boitier (26), peut être aligné avec une ou plusieurs ouvertures de vissage (6) de la plaque d'ostéosynthèse (1).

21. Plaque d'ostéosynthèse selon la revendication 20, **caractérisée en ce que** l'évidement de passage (30, 31) forme un compartiment de réception pour une broche (33), qui est montée en coulissement longitudinal dans le compartiment de réception.

22. Plaque d'ostéosynthèse selon la revendication 21, **caractérisée en ce que** la broche (33) est déplacée dans une position extrême proximale par un ressort (34), et peut coulisser dans la direction distale à l'encontre de l'action de ce ressort (34).

23. Plaque d'ostéosynthèse selon l'une des revendications 20 à 22, **caractérisée en ce que** le boitier (26) est monté rotatif sur le support (25), de manière telle, que pour des positions angulaires différentes, différents compartiments de réception (10, 11) et évidements de passage (30, 31) puissent être alignés avec les ouvertures de vissage (6).

24. Plaque d'ostéosynthèse selon la revendication 23, **caractérisée en ce que** sur le boitier (26) est agencée une poignée de rotation (29).
